Europäisches Patentamt

European Patent Office

Office européen des brevets

(19)

(11) Publication number: **0 255 755**
**A2**

# EUROPEAN PATENT APPLICATION

(12)

(21) Application number: **87304976.1**

(22) Date of filing: **04.06.87**

(51) Int. Cl.⁴: **A 61 K 39/02**
**A 61 K 39/108**

(30) Priority: **05.06.86 US 871025**

(43) Date of publication of application:
**10.02.88 Bulletin 88/06**

(84) Designated Contracting States:
**CH DE GB IT LI SE**

(71) Applicant: **Baylor College of Medicine**
**Texas Medical Center 1200 Moursund Avenue**
**Houston Texas 77030 (US)**

(72) Inventor: **Evans, Doyle J., Jr.**
**5111 Doliver Drive**
**Houston Texas 77056 (US)**

**Graham, David Y.**
**4051 Mischire**
**Houston Texas 77025 (US)**

**Evans, Dolores G.**
**5111 Doliver Drive**
**Houston Texas 77056 (US)**

(74) Representative: **Wilkinson, Stephen John et al**
**c/o Stevens, Hewlett & Perkins 5 Quality Court Chancery**
**Lane**
**London WC2A 1HZ (GB)**

The microorganism(s) has (have) been deposited with American Type Culture Collection under number(s) ATCC 53623, 53624.

(54) **Vaccine preparation.**

(57) Disclosed are bacterial vaccines having their internal DNA's destroyed and their methods of production.

EP 0 255 755 A2

0 255 755

**Description**

< <DPA NB=1> >VACCINE PREPARATION


BACKGROUND OF THE INVENTION

1. Field of the Invention

This invention is directed to the preparation of vaccines especially vaccines for bacterial pathogens and particularly to the preparation of oral whole-cell vaccines.

2. Description of Prior Art

Acute diarrhea is a significant health problem worldwide, particularly for residents of and travellers to less developed nations. Enterotoxigenic Escherichia coli (ETEC) is a major cause of diarrhea in underdeveloped areas (1, 2, 3, 4). Recent work in the search for a vaccine to prevent ETEC-induced diarrhea has focused on purified preparations of the bacterial adhesins, such as the colonization factor antignes, (CFAs) (5, 6, 7) and on oral administration of non-toxigenic CFA-positive ETEC organisms (7, 8). Previous experiments in which purified CFA/I was administered orally to adult rabbits (6, 9) or human volunteers (5) supported the hypothesis that protection against challenge with virulent ETEC was dependent on a CFA-specific intestinal secretory IgA response. This has been further supported by results of experiments in which a non-toxigenic CFA/II+ candidate vaccine strain (E1392-75-2A; 06:H16, CS1, CS3) afforded significant protection against toxigenic ETEC challenge (10).

There is general agreement that maximal protective immunization against ETEC is elicited by natural symptomatic infection, in which case there is a high degree of intimate contact between bacterial antigens and the intestinal immune system. (9, 10, 11)

Vaccine regiments which mimic actual infection with bacterial enteropathogens appear to offer the best chance of success for long-term immunoprotection against naturally-acquired infections caused by enterotoxigenic Eschericia coli (ETEC) or Vibrio cholerae. To date, candidate vaccines employing live but attenuated organisms, bacterial cell preparations lacking possibly essential virulence factors, or bacterial cells killed by heat, chemicals or irradiation have not proven to be practical or generally effective. Potential drawbacks of the above-mentioned vaccines include the risk of reversion to full virulence and diminution of antigenic repertoire.

In most instances actual disease caused by bacteria produces good and lasting immunity. In part, this is because of exposure to numerous bacterial antigens including all of the relevant virulence factors. The ideal vaccine would minic actual infection without risk to the vaccine. All of the currently used procedures for preparing whole-cell bacterial vaccines alter the bacterial cells in some fashion so that the full immunizing capacity of the vaccine is probably not being employed. In many cases it is not known which particular surface antigen, or which combination of antigens, is important to immunoprotection especially since the vaccines employed are imperfect. Production of new vaccine products in the U.S. is practically nil; also many vaccines have been removed from the marked due to newly assessed risks

SUMMARY OF THE INVENTION

The present invention is directed to a novel procedure for preparing and killed (i.e. non-viable) bacterial cells for administration as a vaccine.

The present invention is directed to an oral vaccine which presents ETEC antigens in a natural but non-replicating state. We report here results obtained with an oral whole-cell ETEC vaccine employing virulent (i.e. LT+ST+CFA/I+) bacterial cells rendered nonviolation by a lethal dose of colicin E2. Colicin E2 is a potent DNA endonuclease which is transported into E2-sensitive cells without damaging the cell membrane (12, 13). Colicin E2 specifically destroys the bacterial chromosome and any resident plasmid DNA without disrupting the structural integrity of the bacterial cell. This vaccine is effective in stimulating anti-CFA/I intestinal secretory IgA and on its ability to protect against oral challenge with a high dose of virulent ETEC.

The present invention is also directed to an orally administered vaccine produced from whole bacterial cells of ETEC strain H-10407 (ST+LT+; 078:HII:CFA/I) which has been successfully tested. Bacterial cells were rendered non-viable by treatment with a potent DNA endonuclease, colicin E2. Young healthy adult volunteers were vaccinated orally with priming and booster doses of approximately 3 x $10^{10}$ vaccine cells. In the first experiment, 12 of 13 vaccinated volunteers showed a significant (P < 0.03) increase in intestinal CFA/I-specific secretory IgA. A follow up double-blind placebo controlled trial of 21 volunteers revealed a significant increase in CFA/I-specific intestinal IgA in 10 of 12 vaccines compared to none of 9 who received placebo. Challenge with virulent strain H-10407 (3 x $10^9$ cells) produced diarrhea in 8 of 9 (89%) of the placebo-treated volunteers and 2 of 10 (20%) vaccinated volunteers. There was a definite relationship between the anti-CFA/I mucosal immune response and response to challenge; and 2 vaccinees who became ill had either no or blunted response to vaccination. Other vaccine preparation afforded both a significant intestinal immune response and significant protection against challenge with the virulent organism. Our method of vaccine production is applicable to other bacterial enteropathogens.

Bacterial cells prepared in this manner represent an extremely attractive vaccine because the procedure causes no major alteration of the bacterial surface antigens and structural components important to the immune response. The agent used to kill the bacterial cells in this procedure is chosen for its extreme specificity which permits it to interact only with certain strains of bacteria; consequently the killing agent cannot present a hazard to either the vaccinee or the vaccinee's normal bacterial flora. The preferred killing agents are those commonly produced by strains of bacteria present in the human body. For example, the vaccine strain can be Escherichia coli and the killing agent can be a bacteriocin colicin. According to the present invention, the bacterial cells are killed by the internal destruction of their DNA, both chromosomal and plasmid DNAs, by enzyme activity (DNase). This greatly reduces the possibility of exposing the vaccinee's normal flora to plasmids encoding for bacterial virulence factors. Through genetic engineering, bacterial strains can be made susceptible to the bacteriocin or choice. The bacteria are killed without exposure to heat, radiation or chemicals which denature or alter the configuration of the antigenic components important to the process of immunization.

Bacterial vaccine prepared by the method according to the present invention are antigenically equivalent to the living cells and therefore represent the best possible vaccine other than living bacterial. The method can be employed in conjunction with other technology so that vaccine strains can be genetically engineered; for example, so that lower doses are necessary for protection. Another important feature of the method is that the type of bacteriocin (for example, colicins, pyocins, and vibriocins), in terms of mechanism of action, can be selected to advantage; for example since the vaccine is administered orally and since the vaccine strain contains virulence-encoding plasmids it is advantageous to know that these plasmids are being destroyed by the colicin treatment.

In one embodiment this invention is directed to an oral whole-cell vaccine for the prevention of enterotoxigenic E. coli (ETEC) diarrhea; but the method encompasses the entire range of bacterial diseases. For example, diseases for which pilus (fimbrial) vaccines are potentially useful include whooping cough (B. pertussis), burn infections (Pseudomonas sps), urinary tract infections (E. coli) and gonorrhea (N. gonorrhoeae); and bacterial diseases of domestic animals Enteropathogens for which oral vaccines are being sought include Shigella, Salmonella, E. coli and Vibrio cholerae.

Other potential users include scientists interested in basic research concerning the molecular basis of immunity against bacterial infections and scientists actively involved in developing new vaccines against specific bacterial diseases where no vaccine is currently available or where improved vaccines are being sought because of problems with vaccines prepared by currently used methods. Both types of research will be greatly facilitated since the killed whole-cell vaccine according to the present invention can be produced commercially, on a customized basis, for research purposes.

It is therefore an object of the present invention to provide vaccines and a new method for preparing vaccines.

Another object of the present invention is the provision of vaccines and of a method for preparing vaccines for bacterial pathogens.

Yet another object of the present invention is the provision of oral whole-cell vaccines and a method for preparing oral whole-cell vaccines.

A particular object of the present invention is the provision of a novel procedure for preparing non-viable bacterial cells for administration as a vaccine.

A further object of the present invention is the provision of a vaccine and vaccine preparation method which causes no major alteration of the bacterial surface antigens and structural components important to the immune response.

A still further object of the present invention is the provision of a method of vaccine preparation which utilizes a cell specific bacterial killing agent which does not present a hazard to the vaccinee.

A particular object of the present invention is the provision of such a vaccine and method in which the killing agent is a bacteriocin.

Another object of the present invention is the provision of a vaccine preparation method in which bacterial cells are killed by the internal destruction of their DNA by enzyme activity.

Yet another object of the present invention is the provision of a vaccine preparation method in which bacteria can be killed without exposure to heat, radiation or chemicals which alter antigenic components important to immunization.

Still another object of the present invention is the provision of a vaccine which is antigenically equivalent to the living cells.

A further object of the present invention is the provision of a use for bacteriocins.

To one of ordinary skill in this art who has the benefit of the teachings of this invention, other and further objects, features, and advantages will be clear from the following description of presently preferred embodiments of the invention.

BRIEF DESCRIPTION OF DRAWINGS

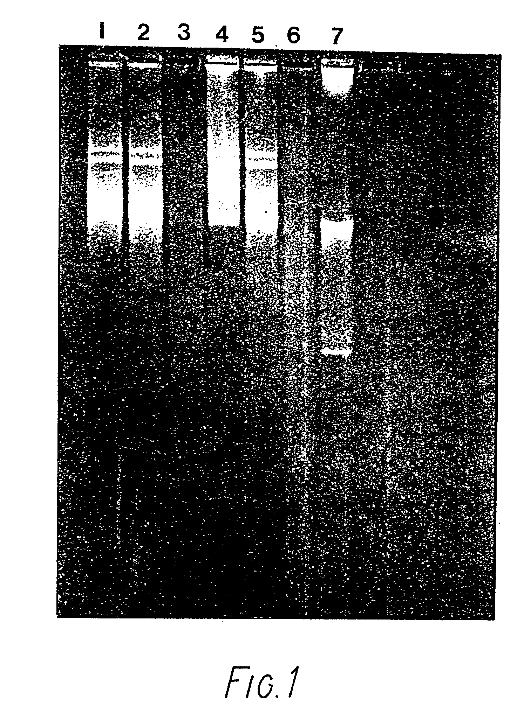

Figure 1 is a SDS-agarose gel with DNA from strain H-10407 cleared lysated stained with ethidium bromide. Lanes 1, 2, 4 and 5 show the DNA pattern of cells before treatment with the DNA endonuclease, colicin E2. Lanes 3 and 6 show the results of exposure of bacterial cells to a lethal dose of colicin E2 confirming that colicin E2 is a potent DNA endonuclease. Lane 7 shows the linear chromosomal DNA and a $3.7 \times 10^6$ molecular weight plasid DNA. Note the absence of linear chromosomal DNA in lanes 3 and 6.

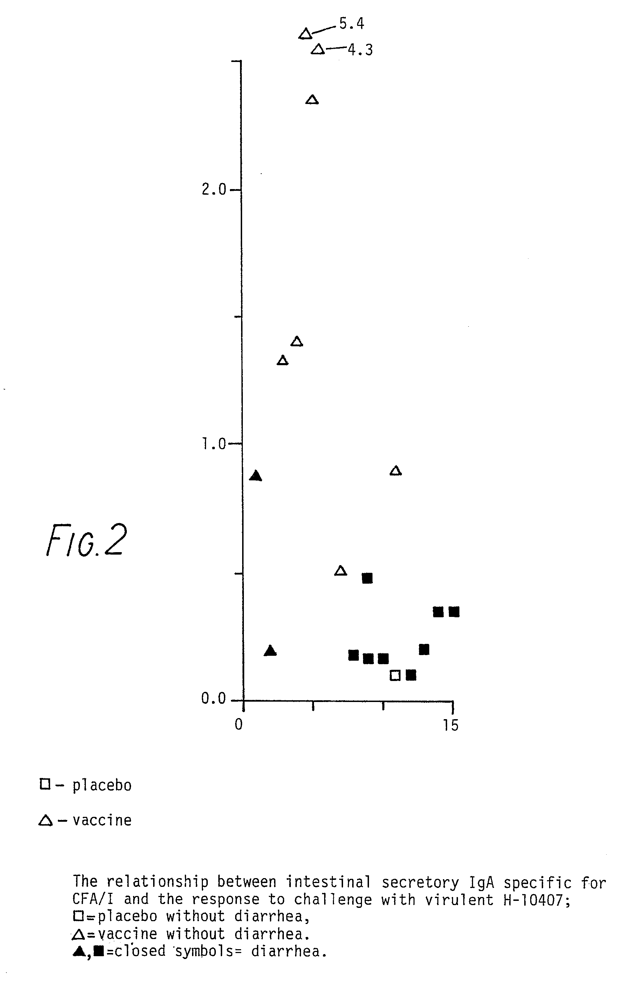

Figure 2 shows the relationship of the percent anti-CFA/I specific secretory IgA with the response to challenge with virulent H-10407.

## DESCRIPTION OF PREFERRED EMBODIMENTS

Bacteriocins:

Bacteriocins are protein substances produced by some bacteria and which kill certain other bacteria. The cells which produce a particular bacteriocin are immune to that bacteriocin but may be sensitive to killing by other bacteriocins.

Many genera of bacteria produce plasmid-encoded proteins, the bacteriocins, which are lethal to closely related bacteria. In theory, bacteriocins play an important role in bacterial antagonism in vivo. The mechanism of action of the majority of bacteriocins does not involve lysis of the target bacteria (12) and even colicins El and la, which kill by depolarizing the cell membrane, do not cause lysis or even leakage of large molecules (12). Besides being a potent DNA endonuclease, colicin E2 does not alter the integrity of the bacterial cell membrane; thus, we chose this colicin for preparation of the vaccine described here. Colicin E3, whose mechanism of action is similar to E2 but with RNAase activity, could also have been used to produce a vaccine although we believe that use of colicin E2 has a distinct advantage bacause the vaccinee's normal flora is not exposed to intact genomic or plasmid DNA from virulent E. coli. Whole cell E. coli rendered non-viable by treatment with colicin E2 retain their normal battery of virulence factors and proved to be an effective vaccine.

Non-replicating Whole Cell Vaccine Preparation

The vaccine was made from enterotoxigenic E. coli H-10407 (078:HII:CFA/I) (14) cells in which DNA had been digested by exposure to a lethal dose of colicin E2. The collicin E2 preparation was prepared from E. coli strain E-279 (obtained as a gift from Dr. Jordan Konisky) (12). Briefly, E. coli strain E-279 was grown in Cye broth (15) culture for 18 hours at 37°C (with shaking at 150 rpm). The supernatant containing the colicin E2 was separated by centrifugation (12,000 x g for 15 minutes) followed by filter sterilization with a 0.2 micron filter (Acrodisc, Gelman Sciences, Inc., Ann Arbor, Michigan).

E. coli strain H-10407 cells were grown in 500 ml CYE broth cultures that had been inoculated with 7.5 ml of an 18 hour culture. The cultures were harvested by centrifugation (6,000 x g for 20 minutes) when the optical density (O.D.) at 640 nm was $0.90 \pm 0.10$ which occurred 4.0 to 4.5 hour post-inoculation. Bacterial cells were resuspended in glass-distilled water and the $O.D._{640}$ adjusted to 1.00-1.05. The cells were then shaken (100 rpm) for 45 minutes at 37°C followed by the addition of 0.1 volume of the colicin E2 preparation. The cells were harvested after 45 minutes by centrifugation at 4,500 x g for 20 minutes and resuspended to the original volume is phosphate-buffered saline, pH 7.2 (PBS). Typical plate-count results prior to E2 treatment were 6.0 x $10^8$ bacterial per ml with no E. coli surviving the colicin treatment. The vaccine dose was made of approximately 3.0 x $10^{10}$ colicin-treated bacterial cells.

Preparation of Placebo

Placebo was prepared by suspending 35 g of cornstarch in 900 ml PBS, with stirring and allowing the suspension to settle for 10 minutes. At that time the unsettled particles in the top 600 ml were decanted. This solution was boiled for 5 minutes and diluted further with PBS to resemble the optical density of the vaccine cell preparation. At the time of administration to volunteers, the placebo suspension was thoroughly stirred by a third party not involved in the study, who also measured 50 ml aliguots for oral administration.

Administration of Vaccine

Approximately 3.0 x $10^{10}$ nonviable strain H-10407 cells were suspended in 50 ml of chilled PBS and administered orally to volunteers 2 minutes after taking 0.5 g sodium bicarbonate and followed 5 minutes later by 1 g of sodium bicarbonate and 8 ounces of water. The booster dose was administered by an identical procedure. Volunteers were then observed for systemic or gastrointestinal side effects.

Collection of Intestinal Secretions

Intestinal secretions were collected using Enterotest (HDC Corp., Mountain View, California) capsules. Each capsule contains a 100 cm piece of "fuzzy" nylon attached to a 50 cm string. After the capsule dissolved, the nylon enters the small bowel; the proximal end is not swallowed and is attached to the bed garments. Volunteers swallowed the capsule the night before sample collection and were instructed not to ingest food from 4 hours prior to swallowing the capsule until after the sample collection. Samples of intestinal contents were immediately frozen -70°C. At the time of use proteolytic enzyme inhibitors were added (per ml): 20 ul of 10 mM phenylmethylsulfonyl fluoride, 100 ul of 1 mg/ml trypsin inhibitor in 50 mM EDTA, and 10 ul of 0.5% sodium azide.

Quantitation of Intestinal IgA

Total IgA and anti-CFA/I IgA in the duodenal samples were quantitated using a biotin-amplified ELISA method as previously reported (16). Briefly, microtiter plate (Linbro Scientific Co., Hamden, Connecticut) wells were coated with affinity-purified goat anti-IgA (alpha-chain specific) at 4.0 ug/ml in bicarbonate buffer (pH 9.5) at 4°C. Wells were blocked with 1% BSA in PBS after two washes with PBS (Microtiter Plate Washer Model 120, Flow Labs McLean, Virginia). Samples of intestinal contents were diluted in 1% normal rabbit serum in PBS containing 0.02% Tween 20 (PBST20) and 50 ul added in triplicate, to the microtiter plate wells. After incubation for 2 hours at 45°C, wells were washed 3 times with PBST20, followed by addition of 50 ul of an optimal dilution of alpha-chain specific biotin-conjugated goat anti IgA prepared in 5% normal rabbit serum. After incubation at 37°C for 1 hour, the wells were washed 4 times with PBST20 and an optimal dilution of alkaline phosphatase-conjugated avidin (Vector Labs, Buringame, California) in 10 mM HEPES buffer plus 0.1 M NaCl was added. The plates were then incubated at room temperature for 1 hour and after 3 washes with PBST20, alkaline phosphatase substrate (paranitrophenyl phosphate, Sigma Chemical Co., St. Louis, Missouri) prepared in diethanolamine HCl buffer (pH 9.8) was added. Color development was recorded at 405 nm using a Titertek MC ELISA reader (Flow labs). All samples from an individual volunteer were assayed at the same time. Each plate also contained samples for a standard curve which consisted of dilutions of purified human myeloma IgA (range 1.0 to 50.0 ng/20 ul).

The above procedure was also used to quantitate anti-CFA/I IgA with the exception that wells were coated with 50 ul of purified CFA/I at 1.0 ug/ml in PBS and at 37°C. Concentrations of IgA in samples were calculated from the standard curves. Intestinal anti-CFA/I IgA is expressed as percent of total IgA.

## Quantitation of Serum Anti-CFA/I IgG

Serum anti-CFA/I IgG was determined by titration of pre-vaccination, one month post-vaccination, and one month-booster sera using the ELISA method as previously described (17). Controls included sera known to be positive, or negative, for anti-CFA/I IgG.

## Challenge Dose of strain H-10407

Escherichia coli, strain H-10407 (serotype 078:K80:H11:CFA/I is a well-characterized enterotoxigenic isolate that originated in Bangladesh and has previously been described as possessing colonization factor antigen/I (CFA/I) and both ST and LT enterotoxins (14, 15). To challenge volunteers, the bacteria were grown for 18 hours in Brain-Heart Infusion broth (Difco Labs, Detroit, Michigan). One-tenth of a milliter of this culture was spread on the surface of a casamino acid-yeast extract agar (CFA agar) (15) plate. After incubation for 4 hours at 37°C, the growth was removed via sterile swabs and suspended in phosphate-buffered saline (PBS). The E. coli cells were diluted with PBS to $5.0 \times 10^9$ organisms/ml by adjusting the optical density according to a standard curve. This suspension was kept cold until immediately prior to use (within 3 hours) when a 1:50 dilution was prepared with PBS. The homogeneity of the inoculum was determined by Gram stain before its use and the precise number of viable bacterial was determined by the plate count method. Subjects were given 0.5 g of sodium bicarbonate in 4 ounces of water, 2 minutes before ingesting the bacterial inoculum. Volunteers then received $5.0 \times 10^9$ viable cells of the E. coli strain H-10407 suspended in 50 ml of sterile PBS, pH 7.2. One g of sodium bicarbonate was administered orally 5 minutes later and subjects remained fasting for an additional 4 hours.

## Challenge of Volunteers with Virulent ETEC

To minimize the chance of cross-contamination, challenge experiments were performed in close confinement at the Baylor General Clinical Research Center. This facility was used to ensure proper collection of data pertinent to stool frequency, stool weight, and other symptoms such as fever, cramps, headache, nausea, and vomiting. These symptoms were recorded during the period of observation. Precaution against transmission of enteric infections was taken using a standardized procedure (18).

Volunteers were over 18 years old and of either sex. Each subject underwent a complete history and physical examination and laboratory screen before entering the study. Volunteers were excluded if they had a history of traveler's diarrhea, if they had resided in a Latin American country, or if they had detectable anti-CFA/I or anti-CFA/II serum antibodies (IgG).

The study was approved by the Institutional Review Boards at the Baylor College of Medicine and the hospitals involved. A consent form was signed by each participant of the study.

Each bowel movement that was passed while in the hospital was collected in a plastic bag using a portable camper's toilet. Upon collection, each stool was weighed (± 0.1 g) and the consistency of each bowel movement was characterized as watery, unformed, or formed and the presence or absence of blood or mucus was recorded. The time of each bowel movement was also recorded. ETEC-induced diarrhea was defined as development of 2 unformed stools in addition to one somatic complaint such as headache, abdominal pain, myalgia, or fever.

## Identification of ETEC in Stool Samples

Stool specimens were collected and analyzed for weight and form as previously described (5). Specimens were placed on ice in vials and transported to the laboratory where McConkey agar plates were streaked for isolation of E. coli. After 18 to 24 hours growth at 37°C, isolated lactose-positive colonies were transferred to CFA agar plates and grown for 18 to 24 hours before exmaination for CFA/I. Colonies were picked solely on the basis of isolation; i.e. not according to size or color intensity. Screening was performed by testing for mannose-resistant HA of bovine erythrocytes and random MRHA-positive colonies were confirmed as CFA/I-positive with CFA/I-specific rabbit serum (15, 19). False positive results with the bovine MRHA test are rare (19) and were not seen in the studies reported here.

Analysis of the Effect of Colicin E2 on Bacterial DNA

The effect of colicin E2 treatment on the DNA content of strain H-10407 cells was evaluated. Aliquots (3.0 ml) of bacterial cells were taken before and 45 minutes after addition of E2. Cells were pelleted by centrifugation for 7 minutes at 6,000 x g at 4°C and then resuspended in 40 mM Tris-acetate -2.0 mM $Na_2EDTA$ buffer, pH 7.9 (buffer E) and the $O.D._{640}$ adjusted to 0.800. Cleared cell lysates were prepared by the method of Kado and Liu (20) by suspending the cells in 3% SDS in 50 mM Tris-HCl, pH 12.6 followed by heating at 55°C for 40 minutes. Electrophoresis in 0.7% agarose slab gels (thickness 0.5 mm) in buffer E at 75 mA, 150 V, for 4 to 6 hours. DNA was visualized with ethidium bromide (0.4 ug/ml) and photographed using a Transilluminator Model C-63 (Ultraviolet Products, Inc.) and an MP-4 Polaroid camera.

The bacteriocin, known as colicin E2, is obtained in the form of an 18-hour culture supernatant fluid. The producer strain is E. coli strain 279; the growth medium is CYE broth prepared as described by Evans, et al.

Bacteria are removed from the spent fluid by high-speed centrifugation, followed by filtration; the resultant E2 preparation is sterile.

The E. coli vaccine strain-10407 (serotype 078:HII:CFA/I) is enterotoxigenic; it contains plasmids encoding for LT and ST enterotoxins and for the fimbrial colonization factor antigen termed CFA/I (Evans, et al). CFA/I is an external structure responsible for bacterial attachment to the epithelial cell lining of the small intestine, where the bacteria multiply and produce toxin with resultant diarrhea. The function of the vaccine is to elicit intestinal anti-CFA/I antibodies to prevent attachment (colonization) and thus prevent diarrheal disease. This protective effect has been demonstrated in experimental animals (Evans, et al). The method described here delivers CFA/I to the intestine, via ingestion by vaccines, in large amounts and entirely in the native form; i.e. not altered by purification procedures or by other factors usually associated with the production of whole-cell vaccines such as heat, chemicals or radiation.

Treatment of the vaccine strain with the bacteriocin is as follows: Logarithmic phase bacteria are obtained by centrifugation from aerated cultures grown at 37°C in CYE broth in a water-bath incubator shaker. Cell density is adjusted to an optical density of approximately 1.0 (1.0-1.05) at 640 nm, equivalent to approximately $2.0 \times 10^8$ bacteria per ml of distilled water. Cells are submitted to starvation by shaking at 37°C for 45 minutes before the colicin preparation is added. The amount of colicin added is 10% of the volume in each container, which is many times the lethal dose for the target strain. This is followed by another 45 minutes of shaking at 37°C. The treated cells are then harvested by centrifugation and resuspended in physiological phosphate-buffered saline at 4°C until administered orally to vaccinees (one 50 ml does contains $10^{10}$ bacterial cells). Killing of the vaccine strain is assessed by inoculation onto appropriate agar medium before the vaccine is approved for use.

IMPORTANCE OF INCUBATION CONDITIONS FOR TREATING E. COLI STRAIN H-10407 WITH COLICINE

Although the growth of E. coli strain H-10407 will cease (i.e. no further rise in optical density of the culture) upon adiditon of E-containing supernatant plate counts on such cultures indicate H-10407 survivors in the range of approximately $1 \times 10^3$ per ml of culture. These survivors, if cultured and tested, prove to be E-sensitive upon re-testing. This lack of 100% killing was overcome by developing the method of pre-incubation of the target (H-10407) cells in the absence of nutrients. The following results show that 100% killing was obtained by pre-incubation of the target cells in distilled water, but not pre-incubation in PBS (phosphate-buffered saline).

Experiment 1: Enhancement of killing of strain H-10407 by pre-incubation in distilled water, as compared to suspension in PBS (phosphate-buffered saline).

Target cells: strain H-10407 at $2 \times 10^8$ cells/ml, harvested from young cultures grown in CYE broth.

Treatment: culture supernatant of strain KE-279 containing silicon E grown 18 hours in CYE broth, and cells removed by centrifugation at 10,000 rpm for 15 minutes, followed by filtration through a 0.2 micron Gelman syringe membrane filter.

Procedure: Resuspend target cells in 10 ml of PBS/H20 mixtures (see below) and shake at 37°C for 60 minutes. Then add 1.0 ml of E-containing supernatant (sterile) and shake for additional 60 minutes. Then harvest cells by centrifugation, resuspend in 5.0 ml PBS and perform plate counts to quantitate surviving cells.

RESULTS: <u>Cells Suspended in</u>    <u>Surviving E. coli per ml</u>

(A)  100% PBS /                    $2.4 \times 10^2$

(B)   50% PBS / 50% water          $2.4 \times 10^2$

(C)   25% PBS / 75% water          $2.0 \times 10^1$

(D)            /100% water         $0.0$


NOTE:  In a similar experiment approximately $5 \times 10^7$ cells of strain H-10407 were exposed to a 1:20 dilution of E-containing supernatant, with similar results, as follows:


<u>Cells Suspended in</u>    <u>Surviving E. coli per ml</u>

(A)  100% PBS /                    $1.05 \times 10^2$

(B)   20% PBS / 80% water          $9.0 \times 10^1$

(C)   10% PBS / 90% water          $2.0 \times 10^1$

(D)            /100% water         $0.0$


TITRATION OF E-CONTAINING SUPERNATANT BY PLATE-COUNT METHOD: KILLING OF E. COLI VACCINE STRAIN H-10407

Procedure: Prepare materials as per methods described elsewhere: (A) sterile E-containing CYE broth supernatant of E. coli strain KE-279; and (B) inoculum of strain H-10407 in the form of an overnight (18 hour) CYE broth culture.

1. Inoculate 500 ml of CYE broth in a 1 liter flask, shake at 37°C and record optical density at 640 nm; harvest cells at O.D. of approximately 0.800 final optical density is not critical to experiment; what is needed is (a) young culture (b) enough yield of cells. Inoculum of this 500 ml culture was done using 8.0 ml of the 18 hour broth culture (12 ml in a 50 ml flask shaking at 37°C).

2. Harvest cells from large culture by centrifugation at 6000 rpm for 20 minutes; resuspend cells in distilled water at an optical density of 1.02 at 640 nm (plate count: $8 \times 10^8$ cells per ml).

3. Place 18 ml of this suspension in each of seven 50 ml flasks; place in water-bath shaker for 60 minutes at 37°C.

4. Add 2.0 ml of E-containing supernatant or dilution thereof to flasks; continue shaking for additional 60 minutes (Note: Every 10 minutes invert each flask by hand after covering top with parafilm; this is to insure exposure of 100% of the inside surface of each flask).

5. After 60 minutes, obtain a 5.0 ml sample from each flask and centrifuge at 10,000 rpm for 15 minutes (guarding against cross-contamination by using sterile screw cap centrifuge tubes). Resuspend each of the seven pellets in 5.0 ml of PBS and prepare 1:10, 1:100, etc., dilutions in PBS: plate 0.05 ml of McConkey agar in triplicate for each dilution.

RESULTS:

Dilutions: Prepared / Final

(in flask)          Survivors per Ml

| | | |
|---|---|---|
| **Undiluted | / 1:10 | none |
| 1:10 | / 1:100 | none |
| 1:100 | / 1:1000 | $2.075 \times 10^3$ |
| 1:$10^3$ | / 1:$10^4$ | $<1 \times 10^7$ |
| 1:$10^4$ | / 1:$10^5$ | $<1 \times 10^8$ |
| 1:$10^5$ | / 1:$10^6$ | $<1 \times 10^8$ |
| 1:$10^6$ | / 1:$10^7$ | $<1 \times 10^8$ |

** Undiluted, filter sterilized culture supernatant (containing colicin E2) contained 1.33 mg protein per ml, as determined by the Lowry protein assay using bovine serum albumin as the standard.

RESULTS

Effect on Colicin E2 on ETEC strain H-10407

Neither chromosomal nor plasmid DNA was observed following ethidium bromide staining of SDS-agarose gels confirming that colicin E2 is a potent DNA endonuclease (12). Exposure of bacterial cells to a lethal dose of colicin E2 also did not result in a significant decrease in optical denisty or hemagglutination (HA) titer (mannose-resistant HA of bovine or human RBCs by CFA/I) nor was unusual clumping or settling present, even after 72 hours at 4°C.

Experiment 1: Assessment of Vaccine Immunogenicity

A group of 13 volunteers were vaccinated orally with $3.0 \times 10^{10}$ colicin E2-killed cells of ETEC strain H-10407 as described above. The same dose was administered as a booster one month later. As a group, precent of the intestinal IgA specific for anti-CFA/I was significantly (P<0.03) increased following initial vaccination and booster vaccination ($0.54 \pm 0.58\%$ vs. $2.01 \pm 2.69$ vs $2.7 \pm 2.2\%$ in the intestinal contents obtained pre-vaccine, and 1 month after both initial vaccination and administration of the booster dose, respectively. The percent of CFA/I specific IgA increased in 12 of the 13 volunteers. Serum anti-CFA/I IgG was also measured and it increased in 3 of the 13.

TABLE 1

Immune response following oral administration of nonviable whole cell ETEC vaccine.

| Subject | % Specific anti-CFA/I IgA | | | Serum anti-CFA/I IgG | | |
|---|---|---|---|---|---|---|
| | Before Vaccine | Post Vaccine | Post Booster | Before Vaccine | Post Vaccine | Post Booster |
| 1 | 0.11 | 2.62 | 6.31 | $\leq$2 | 16 | 32 |
| 2 | 0.40 | 1.23 | 3.17 | $\leq$2 | 4 | 4 |
| 3 | 0.19 | 1.00 | 1.00 | $\leq$2 | 8 | 16 |
| 4 | 0.64 | 1.33 | 1.64 | $\leq$2 | 16 | ND |
| 5 | 0.49 | 0.33 | 1.25 | $\leq$2 | ND | 8 |
| 6 | 1.09 | 1.00 | 1.83 | $\leq$2 | $\leq$2 | $\leq$2 |
| 7 | 0.51 | 0.62 | 0.31 | $\leq$2 | $\leq$2 | $\leq$2 |
| 8 | 0.28 | 5.66 | 5.39 | $\leq$2 | $\leq$2 | $\leq$2 |
| 9 | 0.50 | 1.54 | 1.49 | $\leq$2 | $\leq$2 | $\leq$2 |
| 10 | 0.18 | 0.16 | 1.52 | $\leq$2 | $\leq$2 | $\leq$2 |
| 11 | 0.13 | 0.85 | 2.39 | $\leq$2 | $\leq$2 | $\leq$2 |
| 12 | 2.25 | 9.57 | 7.31 | $\leq$2 | $\leq$2 | $\leq$2 |
| 13 | 0.19 | 0.21 | 1.55 | $\leq$2 | $\leq$2 | $\leq$2 |

Experiment 2: Assessment of Vaccine Efficacy

A second group of 21 volunteers participated in a randomized double-blind placebo-controlled study to determine vaccine efficacy. These volunteers received either vaccine or placebo using the same schedule as above. None of the subjects receiving placebo demonstrated an increase in the percent of intestinal IgA specific for CFA/I (mean percent 0.15 $\pm$ 0.07 before vaccination and 0.21 $\pm$ one month after administration of the booster dose). In contrast, the percent of intestinal IgA specific for CFA/I in the vaccinated group increase significantly from their pre-vaccine level compared to the level obtained following administration of the booster (mean 0.27 $\pm$ 0.2% vs 1.95 $\pm$ 1.7% P < 0.01).

Nineteen of the subjects (9 placebo and 10 vaccinated) were challenged with virulent ETEC, strain H-10407, within 7 to 8 weeks of the booster vaccination. Eight of nine (89%) of the placebo-treated volunteers developed ETEC-induced illness whereas only 2 (20%) of the vaccinated volunteers did (P < 0.01). Of interest, the 2 vaccinated subjects that became ill had either no response to vaccination. The relationship between the response to challenge and the intestinal anti-CFA/I IgA levels is shown in Figure 2. Both the vaccinated and placebo-treated subject excreted ETEC following challenge.

The course of ETEC-associated diarrhea was shortened as volunteers received oral antibiotics soon after they were scored as positive for ETEC-associated diarrhea. Ten of the 11 subjects who were scored as positive for ETEC-associated diarrhea passed watery stools. The median stool volume was 1075 ml (range 402 to 1838 ml) during the 24 hour period that began with passage of the first unformed or watery stool.

## Effect of Vaccination on Serum IgG and Intestinal IgA and Anti-CFA/I

The effect of vaccination on humoral and intestinal immune response was assessed. The first experiment was designed to assess anti-CFA/I intestinal IgA and serum IgG antibody responses following oral administration of non-replicating H-10407 cells given in 2 doses approximately one month apart. The results are shown in Figure 2. Eight of 13 volunteers demonstrated an increase in CFA/I-specific intestinal IgA (expressed as percent of total IgA). Five of the remaining 6 subjects responded after the second dose, showing that a second booster dose was required to produce a high percentage of responders. The results of the second experiment were similar. Six of the 11 vaccinees sampled showed an intestinal anti-CFA/I IgA response after the first dose, with the number increasing to 9 following administration of the booster dose. Serum IgG response proved to be a relatively poor indicator of immunoresponsiveness of the intestinal immune system. The relationship between intestinal IgA and serum IgG anti-CFA/I responses is interesting, considering that both doses of vaccine were administered orally. Complete data (post vaccine, post booster) was available on 22 vaccinated subjects; an increase in anti-CFA/I intestinal IgA and serum IgG was found in 8, 12 had an increase in intestinal IgA but not in serum IgG, 2 showed no increase in either IgA or IgG. No subject that received placebo demonstrated an increase in either anti-CFA/I intestinal IgA or serum IgG.

## Untoward Effects

Only one subject experienced a systemic effect (such as fever, malaise, headache, rash, etc.) following vaccination. The patient complained of dizziness 1 hour after receiving the vaccine which was relieved by eating. Six subjects experienced loose stools after dosing, 3 in the placebo group and 3 of the total group of subjects who received vaccine. This was characterized as: #1 (vaccine) had several loose stools 1 day following booster, #2 (vaccine) had loose stools for 4-5 days following primary vaccine dose, #3 (vaccine) had loose stools for 3 days following the primary vaccination and on the day of the booster had 5-7 watery stools, #4 (placebo) had loose stools (without change in stool frequency) after both the primary placebo vaccine and the booster, #5 (placebo) had one day of loose stool following the placebo booster, and #6 (placebo) had a few loose stools on the day of the primary placebo vaccine and stools of looser consistency for 5 days following the placebo booster (with no increase in stool frequency).

ETEC adhere to the intestinal mucosa via specific fibrial colonization factor antigens. Theoretically ETEC-induced diarrhea could be prevented by blocking adherance of the bacteria to the intestinal epithelium and there is solid experimental evidence to support this idea (6, 8, 10). Current evidence suggests that the local immune system is of major importance in preventing diseases caused by pathogens which initially colonize the gastrointestinal or respiratory mucosa (21, 22). The protective role of the mucosal immune system, particularly the secretory (IgA (sIgA) immune response, has been demonstrated for both viruses such as parainfluenzae type 1 (23) and poliovirus (24) and for some bacterial enteropathogens (21, 25).

ETEC-induced diarrhea elicits humoral and intestinal immune responses and homologous protection. (7, 8, 26) Immune responses have been demonstrated to occur against various ETEC virulence factors such as LT, ST, CFA/I or CFA/II. (8, 27) To date, experimental vaccines designed to protect against bacterial enteropathies such as cholera or ETEC-induced diarrhea have either failed to protect, have demonstrated low efficacy, or have produced an unacceptable rate of side effects such as diarrhea (10). Purified cholera toxoid alone has not been an effective immunogen, independent of the route of administration (8). The efficacy of attenuated V. cholerae strains as vaccines has either been low or has caused mild to serious diarrhea in at least 25% of the vaccines (5, 10, 28). Attempts to protect against ETEC-induced diarrhea by oral administration of purified CFA/I or CFA/II have also been unsuccessful although promising results were obtained with animal models (6, 29). In a previous experiment we demonstrated that a subcutaneous priming dose of purified CFA/I followed by oral boosting, resulted in an increased intestinal anti-CFA sIgA response in 4 of 8 volunteers. The volunteers demonstrating the increase in specific sIgA were also protected against challenge with homologous ETEC (50). Recently a vaccine consisting of an attenuated CFA/II-producing ETEC strain lacking both LT and ST (7) induced significant protection in vaccinees but again with an unacceptable degree of diarrhea caused by the vaccine (10).

The success achieved in attaining significant rise in both intestinal anti-CFA/I IgA levels and protection against challenge with live homologous ETEC answered our initial questions concerning the safety and efficacy of oral whole cell anti-ETEC vaccines prepared by the colicin method described here. In the challenge study, a dose of strain H-10407 which produced illness in 8 of 9 (89%) of the placebo-treated controls caused diarrhea in only 2 of 10 (20% of the vaccinees and these 2 vaccinees were among the 3 who also demonstrated a poor local immune response to the vaccine. Intestinal IgA might not be the only protective immune response but, unlike serum IgG, does prove to be an excellent predictor of such a response. In these studies we did not measure intestinal antibody responses to ST, LT or somatic antigen, but it can be reasonably speculated that these antigens could account, in part, for the observed protective effect of the vaccine. We believe that our results strongly support the approach of administering vaccines against bacterial enteropathogens by the oral route and in a form which most closely resembles live, virulent, organisms. For example, fimbriae such as CFA/I could perform their biological function (adherence to host intestinal epithelial cell receptors) with much greater efficiency when presented in their natural state on the bacterial cell surface than when presented as individual macromolecules. Likewise, the local intestinal immune system might be more responsive to fimbriae presented in their natural array, which mimics replicate rather than non-replicative antigen. The results we

**0 255 755**

obtained in terms of the fate of the challenge strain in the vaccinees, versus the placebo-treated controls, is consistent with our hypothesis that one of the major mechanism of protection is prevention of colonization. Protection was unlikely due to bactericidal antibody as all of the vaccinees and placebo-treated subjects, irrespective of illness, excreted large numbers of the challenge organism. Nevertheless, the role of antigens other than CFA/I in immunoprotection can now be tested using the colicin method of vaccine preparation in conjunction with vaccine and/or challenge ETEC of homologous or heterologous serotype.

According to the present invention, the bacterial vaccine strain possesses external antigens (either somatic, pilus, fimbrial, capsular, etc.) which are believed to give rise to protective antibodies. The present invention can be used with any bacterial enteropathogen and with any whole-cell bacterial vaccine now employing bacteria killed by other methods in which surface antigens are important to the immunization. Also, the present invention can be used for vaccines for domestic animals, including enterotoxigenic E. coli which possess animal-specific colonization factor antigens such as K88 and K99 antigens and a bacteriocin which is both highly lethal to the vaccine strain and acts without destroying the integrity of the bacterial cells or their surface antigens.

Due to the specificity of bacteriocins, each target or vaccine strain can be matched with an appropriate bacteriocin and treatment conditions adjusted accordingly. For example, for Vibrio cholerae a vibriocin with the appropriate mechanism of action is used. A large variety of vibriocin-producing vibrios are readily available. Advantageously, the present invention is applicable to all bacterial vaccines and methods of their production.

In conclusion, therefore, it is seen that the present invention and the embodiments disclosed herein are well adapted to carry out the objectives and obtain the ends set forth as well as others inherent therein. One of skill in this art will appreciate that changes can be made in the processes and methods of the invention without departing from its spirit and its scope as defined by the following claims.

The micro-organism identified as ETEC Strain H-10407 (ST + LT+; 078: HLL: CFA/I) was deposited with the American Type Culture Collection (ATCC), 12301 Parklawn Drive, Rockville, Maryland 20852, USA on 27th May 1987 and has been given ATCC No. 53623

The micro-organism identified as E. Coli Strain E-279 was deposited with the ATCC on 27th May 1987 and has been given ATCC No. 53624.

REFERENCES

1. Moseley SL, Echeverria P, Seriwatana J, et al. Identification of enterotoxigenic Escherichia coli by colony hybridization using three enterotoxin gene probes. J Infect Dia 1982;145:863-9.

2. Merson JH, Morris GK, Sack DA, et al. Travelers' diarrhea in Mexico: a prospective study of physicians and family members attending a congress. N Engl J Med 1976;294:1299-1305.

3. Black RE, Brown KH, Becker S, Alim Abdul ARM, Huq I. Longitudinal studies of infectious diseases and physical growth of children in rural Bangladesh. II. Incidence of diarrhea and association with known pathogens. Am J Epidemiol 1982;115:315-24.

4. Back E, Mollby R, Kaijser B, Stintzing G, Wadstrom T, Habte D. Enterotoxigenic Escherichia coli and other gram-negative bacteria of infantile diarrhea: surface antigens, hemagglutinins, colonization factor antigen, and loss of enterotoxigenicity. J Infect Dis 1980; 142:318-27.

5. Evans DG, Graham DY, Evans DJ, Jr. Administration of purified colonization factor antigens (CFA/I, CFA/II) of enterotoxigenic Escherichia coli to volunteers. Response to challenge with virulent enterotoxigenic Escherichia coli. Gastroenterology 1984;87:934-40.

6. Evans DG, Cabada FJ, Evans DJ, Jr. Correlation between intestinal immune response to colonization factor antigen/I (CFA/I) and acquired resistance to enterotoxigenic Escherichia coli diarrhea in an adult rabbit model. Eur J Clin Microbiol 1982;1:178-85.

7. Levine MM. Travellers' diarrhea: prospects of successful immunoprophylaxis. Scand J Gastroenterol 1983;18(Suppl 84):121-134.

8. Levine MM, Kaper JB, Black RE, Clements ML. New knowledge on pathogenesis of bacterial enteric infections as applied to vaccine development. Microbiol Rev 1983;47:510-50.

9. Cabada FJ, Evans DG, Eans DJ, Jr. Immunoprotection against enterotoxigenic Escherichia coli diarrhea in rabbits by peroral administration of purified colonization factor antigen/I (CFA/I). FEMS Microbiol Lett 1981;11:303-7.

10. Candy DCA. New enteric vaccines: application of new knowledge of receptors and recognition in enteric infections. Trans N Soc Trop Med Hyg 1985;79:577-80.

11. Germainier R. Oral vaccination against enteric bacterial infections: an overview. Infection 1984;12:138-42.

12. Konisky J. Colicins and other bacteriocins with established modes of action. Ann Rev Microbiol 1982;36:125-44.

13. Shannon R, Hedges AJ. Reversibility of the specific adsorption of colicin E2-P2 to cells of colicin-sensitive strains of Escherichia coli. J Bacteriol 1973;116:1136-44.

14. Evans DG, Silver RP, Evans DJ, Jr., Chase DG, Gorbach SL. Plasmid-controlled colonization factors associated with virulence in Escherichia coli enterotoxigenic for humans. Infect Immune 1975;12:656-67.

15. Evans DG, Evans DJ, Jr., Tjoa W. Hemagglutination of human group A erythrocytes by enterotoxigenic Escherichia coli isolated from adults with diarrhea: correlation with colonization factor. Infect Immun 1979;18:330-7.

11

16. Adler-Storthz K, Dreesman GR, Graham DY, Evans DG. Biotin-avidin amplified ELISA for quantitation of human IgA. J Immunoassay 1985;6:67-77.

17. Clegg S, Evans DG, Evans DJ, Jr. Enzyme-linked immunosorbent assay for quantitating the humoral immune response to the colonization factor antigen/I of enterotoxigenic Escherichia coli. Infect Immun 1980;27:525-31.

18. Graham DY, Estes MK, Gentry LO. Double-blind comparison of bismuth subsalicylate and placebo in the prevention and treatment of enterotoxigenic Escherichia coli-induced diarrhea in volunteers. Gastroenterology 1983;85:1017-22.

19. Evans DJ, Jr., Evans DG, Young LS, Pitt J. Hemagglutination-typing of Escherichia coli. Definition of seven hemagglutination types. J Clin Microbiol 1980;12:235-42.

20. Kado CI, Lui ST. Rapid procedure for detection and isolation of large and small plasmids. J Bacteriol 1981;145:1365-73.

21. Fubara ESS, Freter R. Protection against enteric bacterial infection by secretory IgA antibodies. J Immunol 1973;111:395-402.

22. Cebra JJ, Kamat R, Gearhart R, Robertson SM, Tseng J. The secretory IgA system of the gut. In: Immunology of the gut. Ciba Foundation Symposium No. 48. London:Pitman, 1977:5-28.

23. Smith CB, Purcell RH, Ballanti JA, Chanock RM. Protective effect of antibody to parainfluenzae type 1 virus. N Engl J Med 1966;275:1145-58.

24. Ogra PL, Karzon DT, Rightland F, McGilliuray M. Immunoglobulin response in serum and secretions after immunization with live and inactivated poliovaccine and natural infection. N Engl J Med 1968;279:839-900.

25. Gindrat JJ, Gothefors L, Hanson LA, Winberg J. Antibodies in human milk against E. coli of the serogroups most commonly found in neonatal infections. Acta Paediatr Scand 1972;61:587-90.

26. Evans DG, Evans DJ, Jr. Colinization factor antigens of human associated enterotoxigenic Escherichia coli. In: Robbins JB, Hill JC, Sadoff JC, eds. Bacterial Vaccines. New York: Thieme-Stratton, Inc., 1982:104-12.

27. Levine MM, Ristaino P, Parley G, et al. Coli surface antigens 1 and 3 of colonization factor antigen II-positive enterotoxigenic Escherichia coli: morphology, purification and immune responses in humans. Infect Immun 1984;44:409-20.

28. Levine MM, Black RE, Clements ML, et al. Texas Star-SR: attenuated Vibrio cholerae oral vaccine candidate. Dev Biol Stand 1983;53:59-65.

29. Boedeker EC, Young CR, Collins HH, et al. Toward a vaccine for travelers' diarrhea: mucosally-administered E. colicolonization factor antigens elicit a specific local immunoglobulin A response in isolated intestinal loops. Gastroenterology 1982;82:1020

## Claims

1. A method of preparing DNA destroyed bacterial cells comprising the steps of:
starving the bacterial cells:
then killing the bacterial cells by internal destruction of their DNA with DNAase;
further starving the killed bacterial cells;
harvesting the starved and killed bacterial cells;
suspending the harvested cells in a physiological buffer; and
assessing the effectiveness of the destroyed bacterial cells before utilizing the destroyed bacterial cells.

2. The method of Claim 1 wherein,
the bacterial cells are starved and then killed at a temperature 37°C; and
the bacteria is Escherichia coli (serotype 078:HII:CFA/I).

3. The method of Claim 2 wherein the DNAase is selected from a group consisting of colicin E2, E3, pyocin AP41 and vibriocin.

4. A bacterial vaccine prepared by the method of Claim 1 effective to induce immunological response when a pharmacological amount of the bacterial vaccine is administered orally.

5. An enteropathogen bacterial vaccine consisting essentially of bacterial cells having internally destroyed DNA with a strain specific DNAase.

6. The bacterial vaccine of Claim 5 wherein,
the bacteria is Escherichia coli (serotype 078:HII:CFA/I), and
the DNAase is colicin E2.

7. A bacterial vaccine for CFA/I consisting essentially of:
bacteria killed by DNAase suspended in a physiological solution for oral administration,
the vaccine containing sufficient killed bacteria to induce immunological response on oral administration of a pharmacological quantity thereof.

8. A bacterial vaccine for CFRA/II consisting essentially of a bacterial with a CFA/II antigen;
the DNA of the bacteria having been killed in DNAase;
the killed bacteria being suspended in a physiological solution for oral administration;

said vaccine containing sufficient killed bacteria to induce immunological response on oral administration of a pharmacological quantity thereof.

9. A method of preparing a bacterial vaccine comprising,
destroying DNA of bacterial cells of the vaccine.

10. The method of Claim 11 where,
the DNA is destroyed by a DNAase specific to bacteria of the bacterial vaccine.

11. The method of Claim 11 where,
the DNA is destroyed by DNAase selected from the group consisting of colicin E2, E3, pyocin AP41 and vibriocin.

12. A vaccine effective against enterotoxigenic Escherichia coli (serotype 078:HII:CFA/I) comprising,
DNAase killed Escherichia coli.

13. A bacterial vaccine including,
bacteria cells having their internal DNA destroyed.

14. A bacterial vaccine including,
bacteria cells having their internal DNA destroyed by DNAase.

0255755

FIG.1

0255755

□ – placebo

△ – vaccine

The relationship between intestinal secretory IgA specific for CFA/I and the response to challenge with virulent H-10407; □=placebo without diarrhea, △=vaccine without diarrhea. ▲,■=closed symbols= diarrhea.